(19) Europäisches Patentamt

European Patent Office

Office européen des brevets

(11) **EP 1 643 241 A2**

(12) **EUROPÄISCHE PATENTANMELDUNG**

(43) Veröffentlichungstag:
**05.04.2006 Patentblatt 2006/14**

(51) Int Cl.:
***G01N 27/12*** *(2006.01)*      ***G01N 33/00*** *(2006.01)*

(21) Anmeldenummer: **05107033.2**

(22) Anmeldetag: **29.07.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **30.09.2004  DE 102004047466**

(71) Anmelder: **ROBERT BOSCH GMBH**
    **70442 Stuttgart (DE)**

(72) Erfinder:
• **Brinz, Thomas**
   **73266, Bissingen A.D. Teck (DE)**
• **Mirsky, Vladimir**
   **93059, Regensburg (DE)**
• **Wolfbeis, Otto**
   **93051, Regensburg (DE)**
• **Jockel, Jörg**
   **70839, Gerlingen (DE)**
• **Hao, Qingli**
   **93051, Regensburg (DE)**

(54) **Verfahren und Sensor zur Bestimmung von sauren Gasen enthaltend eine gassensitive Polymerschicht**

(57)    Es wird ein Verfahren und ein Sensor zur Bestimmung saurer Gasen in Gasgemischen beschrieben, wie insbesondere Chlorwasserstoff, Schwefeldioxid oder Stickstoffdioxid. Dabei wird eine Polymerschicht (20), die ihre elektrische Leitfähigkeit unter Einwirkung des zu bestimmenden Gases verändert, dem Gasgemisch ausgesetzt und die elektrische Leitfähigkeit, der elektrische Widerstand oder die Impedanz der Polymerschicht (20) bestimmt. Zur Desorption des zu bestimmenden Gases wird in bestimmten Zeitabständen eine Erhitzung der Polymerschicht (20) auf eine gegenüber der Umgebungstemperatur erhöhte Temperatur durchgeführt.

Fig. 1

EP 1 643 241 A2

**Beschreibung**

Stand der Technik

Die Erfindung bezieht sich auf ein Verfahren und einen Sensor zur Durchführung desselben sowie auf deren Verwendung gemäß dem Oberbegriff der unabhängigen Ansprüche.

Es ist bekannt, dass insbesondere bei Kabelbränden größere Mengen an gasförmigem Chlorwasserstoff freigesetzt werden können, die zu gesundheitlichen Schäden führen. Es besteht somit ein Bedarf an geeigneten Sensoren, die auch kleinere Mengen an freigesetztem Chlorwasserstoff oder anderen sauren Verbrennungsgasen wie Schwefeldioxid oder Stickoxide zuverlässig erfassen können und somit eine frühzeitige Detektion von Bränden ermöglichen.

So ist beispielsweise aus der BR 9105139 A ein Sensor zur Bestimmung von Chlorwasserstoff auf der Basis einer sensitiven Schicht aus Polyanilin bekannt. Dieses System zeigt jedoch nach Beaufschlagung mit Chlorwasserstoff bei Raumtemperatur ein unzureichendes Rückstellungvermögen, wodurch ein Einsatz dieses Sensors als Brandmelder unmöglich wird.

Der vorliegenden Erfindung liegt die Aufgabe zugrunde, ein Verfahren sowie einen Sensor zur Bestimmung saurer Gase bereitzustellen, die eine genaue Detektion derselben ermöglichen, ohne die Nachteile der Sensoren gemäß Stand der Technik aufzuweisen.

Vorteile der Erfmdung

Das erfindungsgemäße Verfahren bzw. der erfindungsgemäße Sensor hat den Vorteil, dass die der Erfmdung zugrundeliegende Aufgabe in vorteilhafter Weise gelöst wird. Dabei wird auf eine sensitive Schicht zurückgegriffen, deren elektrische Eigenschaften sich bereits bei Kontakt mit geringsten Mengen eines zu bestimmenden sauren Gases merklich verändern, wobei die Anlagerung des zu bestimmenden Gases reversibel ist. Zur Desorption des zu bestimmenden Gases erfolgt in bestimmten Zeitabständen eine Erhitzung der sensitiven Schicht auf eine gegenüber der Umgebungstemperatur erhöhte Temperatur. Auf diese Weise ist eine einfache Regenerierung der sensitiven Schicht gewährleistet.

Mit den in den Unteransprüchen aufgeführten Maßnahmen sind vorteilhafte Weiterbildungen des erfindungsgemäßen Verfahrens bzw. Sensors möglich.

So eignet sich als sensitive Schicht insbesondere eine Schicht eines Polymers mit basischen funktionellen Gruppen, wie beispielsweise Polyanilin. Dieses Polymer zeigt eine hohe Affinität zu sauren Gasen wie Chlorwasserstoff u.a..

In einer weiteren vorteilhaften Ausführungsform der vorliegenden Erfindung wird die elektrische Leitfähigkeit bzw. der elektrische Widerstand oder die Impedanz der Polymerschicht mittels zweier Elektroden ermittelt, die in Kontakt mit der Polymerschicht stehen. Dabei wird ein zwischen den Elektroden auftretender Stromfluss oder eine zwischen den Elektroden anliegende Spannung gemessen.

In einer besonders vorteilhaften Ausführungsform der vorliegenden Erfmdung erfolgt periodisch eine Erhitzung der Polymerschicht vorzugsweise auf eine erhöhte Temperatur von 60 bis 400°C, sodass gegebenenfalls angelagerte saure Gase desorbieren.

Der Sensor gestattet die Bestimmung von sauren Gasen im ppb-Bereich, ohne nennenswerte Querempfindlichkeiten zu Wasserdampf oder Kohlendioxid zu zeigen. Er zeichnet sich weiterhin durch eine monatelange Langzeitstabilität aus.

Zeichnung

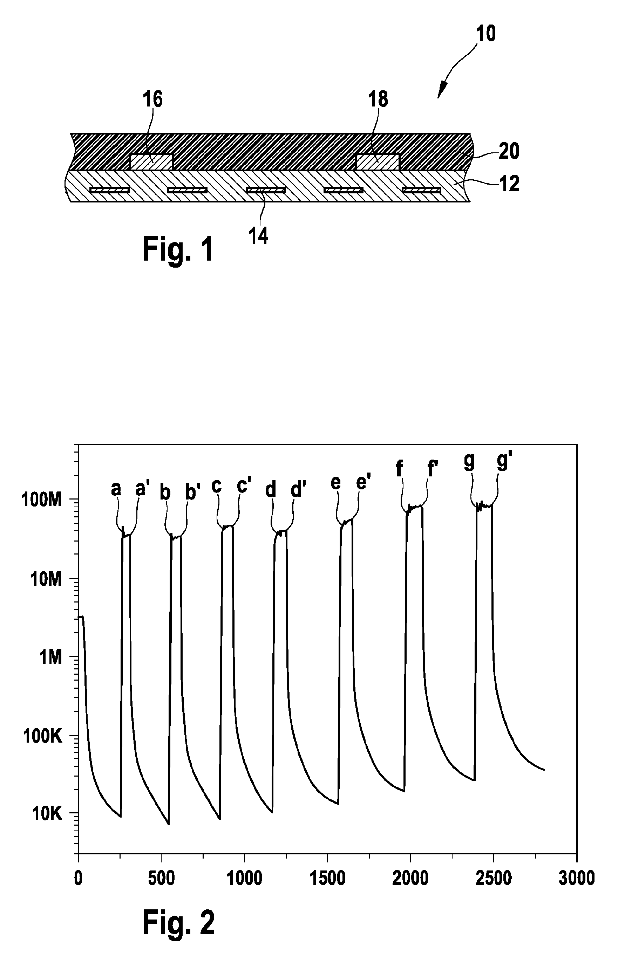

Figur 1 zeigt eine schematische Darstellung eines Ausführungsbeispiels eines Sensors wie er dem erfmdungsgemäßen Verfahren zugrunde liegt. Figur 2 zeigt das Ergebnis einer Messung, bei dem ein Sensor gemäß Figur 1 mit Chlorwasserstoff beaufschlagt wurde.

Ausführungsbeispiel

Das erfindungsgemäße Verfahren zur Bestimmung saurer Gase in Gasgemischen greift insbesondere auf einen Sensor zurück, wie er beispielsweise in Figur 1 schematisch dargestellt ist. Unter einem sauren Gas werden Gase verstanden, die in wässriger Lösung zu einer sauren Reaktion führen. Diese sind beispielsweise Gase wie Halogenwasserstoffe, Schwefeloxide, Blausäure, Stickoxide oder Dämpfe saurer Oxide verstanden.

Der in Figur 1 dargestellte Sensor 10 umfasst ein Substrat 12, das vorzugsweise aus einem Halbleitermaterial, wie beispielsweise Silicium, besteht. Das Substrat 12 weist eine Heizvorrichtung 14 auf, die in Figur 1 schematisch dargestellt ist. Auf der Großfläche des Substrats 12 sind mindestens zwei Messelektroden 16, 18 aufgebracht, die als Interdigitalelektroden ausgeführt sein können. Die Messelektroden 16,18 sind mit einer sensitiven Schicht 20 vorzugsweise überdeckt. Die sensitive Schicht 20 ist aus einem polymeren Material ausgeführt, das bei Kontakt mit einem sauren Gases seine elektrische Leitfähigkeit bzw. seinen elektrischen Widerstand verändert.

Als polymeres Material der sensitiven Schicht 20 eignet sich insbesondere Polyanilin oder ein Polyanilincopolymer, das sich beispielsweise durch Copolymerisation von Anilin mit Anilinderivaten wie Aminobenzoesäuren oder Aminobenzolsulfonsäuren gewinnen lässt. Als Anilinderivate sind insbesondere 2-Aminobenzoesäure, 3-Aminobenzoesäure, 4-Aminobenzoesäure oder 3-Aminobenzolsulfonsäure geeignet. Die Polymerisation wird vorzugsweise elektrochemisch durchgeführt. Dabei wird beispielsweise die Erzeugung der Anilincopolymere potentiostatisch bei einer Spannung von +800 bis +1000 mV gegenüber einer Standard-Kalomelelektrode (SCE) durchgeführt, die Polymerisation von Anilin kann neben diesem Verfahren auch galvanostatisch bei einer Stromdichte von ca. 20 -50 A/m$^2$ oder potentiodynamisch gewonnen werden, wobei bei der potentiodynamischen Methode ein Spannungsbereich von ca. - 20 mV bis +1.0 V abgefahren wird bei einer Variationsgeschwindigkeit von ca. 10 - 30 mV/s. Als Elektrolyt für die elektrochemische Polymerisation wird eine 0.1 molare Monomerlösung vorgelegt, die zusätzlich vorzugsweise einen 01. molaren Gehalt an Schwefelsäure aufweist.

Zur Bestimmung der Konzentration des zu messenden sauren Gases im Gasgemisch wird an die Messelektroden 16, 18 eine konstante elektrische Spannung angelegt und der elektrische Widerstand bzw. die elektrische Leitfähigkeit des sensitiven Schicht 20 bestimmt. Alternativ ist es auch möglich, anstatt einer Gleichspannung den Messelektroden 16, 18 eine Wechselspannung aufzuprägen und die sich zwischen den Messelektroden 16, 18 einstellende Impedanz zu bestimmen. Eine dritte Möglichkeit ist darin zu sehen, an die Messelektroden 16, 18 einen konstanten Strom anzulegen und die sich an den Messelektroden 16, 18 einstellende Spannung zu bestimmen. Als Maß für die Konzentration des zu bestimmenden Gases im Gasgemisch wird die Änderung der elektrischen Leitfähigkeit, des elektrischen Widerstandes bzw. der Impedanz ggf. pro Zeiteinheit herangezogen.

Eine Alternative besteht darin, vier Messelektroden vorzusehen und die Bestimmung des elektrischen Widerstandes der sensitiven Schicht 20 nach der Methode einer Vierpunkt-Messung durchzuführen. Dabei sind vier Messelektroden vorgesehen, wobei eine Paar äußerer Messelektroden ein Paar innerer Messelektroden umgibt bzw. flankiert. Die äußeren Messelektroden sind gegenüber den inneren Messelektroden, sowie die inneren Messelektroden untereinander durch Streifen von Material der sensitiven Schicht beabstandet zueinander angeordnet. Zur Bestimmung des elektrischen Widerstandes der sensitiven Schicht wird an die äußeren Messelektroden eine Spannung von bspw. 50 mV angelegt und der zwischen den äußeren Messelektroden fließende Strom bestimmt. Des weiteren wird der an den inneren Messelektroden dabei auftretende Spannungsabfall hochohmig gemessen. Der elektrische Widerstand der sensitiven Schicht wird erhalten, indem der an den inneren Messelektroden auftretende Spannungsabfall durch den zwischen den äußeren Messelektroden fließende Strom dividiert wird. Dies ermöglicht eine Bestimmung des elektrischen Widerstandes der sensitiven Schicht unter Ausblendung des zwischen Elektrodenmaterial und Polymermaterial der sensitiven Schicht auftretendem Kontaktwiderstand. Polarisationseffekte können vermieden werden, wenn anstatt einer Gleichspannung eine Wechselspannung mit einer Frequenz von 0.5 — 50 Hz angelegt wird.

Bei Kontakt mit einem sauren Gas geht das Polyanilin der sensitiven Schicht 20 von einem neutralen, ungeladenen Zustand, der einer Emeraldine-Base entspricht, in einen protonierten, geladenen und somit elektrisch leitfähigen Zustand über. Dies führt zu einer Abnahme des elektrischen Widerstandes um einen Faktor von ca. 10$^3$. Bei Raumtemperatur ist die Chemisorption des zu bestimmenden sauren Gases jedoch weitgehend irreversibel; es wurde jedoch herausgefunden, dass eine Desorption von sauren Gasen bei höheren Temperaturen möglich ist. Dazu wird der Sensor 10 mittels dem Heizelement 14 zu bestimmten Zeitpunkten auf eine höhere Temperatur gebracht und vorzugsweise für einen bestimmten Zeitraum auf dieser erhöhten Temperatur gehalten. Die Erhitzung des Sensors 10 erfolgt entweder bedarfsweise, beispielsweise bei Sättigung der sensitiven Schicht 20 mit einem zu bestimmenden Gas, oder periodisch. Dabei können Zeitintervalle von 30 Minuten bis 12 Monate, insbesondere zwischen einem und 30 Tagen zwischen den einzelnen Regenerierungsvorgängen gewählt werden. Zur Regenerierung wird der Sensor 10 vorzugsweise auf eine Temperatur zwischen 60 bis 400°C, insbesondere zwischen 100 und 180°C erwärmt. Um die Regenerierung der sensitiven Schicht 20 bedarfsgerecht realisieren zu können, kann der Sensor 10 weiterhin ein nicht dargestelltes Temperaturmessgerät aufweisen, das der Bestimmung der Temperatur der sensitiven Schicht dient.

Der minimale Zeitraum $t_{min}$ in Millisekunden, für den das Sensorelement zu einer vollständigen Desorption des sauren Gases auf der erhöhten Temperaturen gehalten werden muss, ergibt sich nachfolgender Formel (1):

$$t_{min} = \exp(a/T) \tag{1}$$

Dabei ist a eine Zahl zwischen 3000 und 4000, insbesondere zwischen 3500 und 3800 und T die erhöhte Temperatur in Kelvin.

Vor einer ersten Inbetriebnahme des Sensors 10 sollte vorzugsweise eine erste Regenerierung erfolgen.

In einer alternativen Ausführungsform besteht die Möglichkeit, eine Vielzahl von Sensoren auf einem Array anzuordnen, sodass mehrere saure Gase simultan bestimmt werden können. Die einzelnen Sensoren unterscheiden sich dann hinsichtlich der Ausführung ihrer sensitiven Schicht oder hinsichtlich einer auf die sensitive Schicht aufzubringenden Schutzschicht, die beispielsweise auf bestimmte Gase selektive Absorptionsmaterialien aufweist.

In Figur 2 ist eine mittels dem in Figur 1 dargestellten Sensor 10 erhaltene Messkurve dargestellt. Diese entspricht eine Auftragung des elektrischen Widerstandes in Ohm (k = Kiloohm, M = Megaohm) in logarithmischer Darstellung über der Zeit in Sekunden.

Verwendet wurde eine sensitive Schicht aus Polyanilin, die mit einem geeigneten Puffer, beispielsweise mit einer 0.2 molaren Lösung eines Hydrogencarbonat-/Carbonatpuffers, vorbehandelt wurde. Die Vorbehandlung wird vorzugsweise für einen Zeitraum von 10 Minuten bis 12 Stunden durchgeführt. Diese Vorbehandlung dient dem Austausch von im Polymer vorhandenem Sulfat durch Carbonat, welches bei einer ersten Wärmebehandlung als Kohlendioxid entfernt wird.

Die sensitive Schicht wurde jeweils für mehrere Minuten einer Gasatmosphäre mit 80 ppm Chlorwasserstoff ausgesetzt bevor die Schicht durch Erhitzen auf 150°C für 1 bis 2 Minuten regeneriert wurde. Es ist zu erkennen, dass die Regenerierung zu den Zeitpunkten a, b, c, d, e, f, g zu einer starken Zunahme des elektrischen Widerstandes des sensitiven Schicht 20 führt, währendhingegen nach Beendigung der Regenerierung zu den Zeitpunkten a', b', c', d', e', f, g' eine Abnahme des elektrischen Widerstandes der sensitiven Schicht 20 durch Adsorption des zu bestimmenden sauren Gases zu beobachten ist.

Das beschriebene Verfahren zur Bestimmung saurer Gase eignet sich insbesondere zur Früherkennung von Schwel- oder Kabelbränden und somit zur Anwendung in Brandmeldern oder der Bestimmung der Konzentration sauerer Gase in Müllverbrennungsanlagen.

## Patentansprüche

1. Verfahren zur Bestimmung von sauren Gasen in Gasgemischen, wie insbesondere Chlorwasserstoff, Schwefeldioxid oder Stickstoffdioxid, wobei eine Polymerschicht (20), die ihre elektrische Leitfähigkeit unter Einwirkung des zu bestimmenden Gases verändert, dem Gasgemisch ausgesetzt wird, wobei die elektrische Leitfähigkeit, der elektrische Widerstand oder die Impedanz der Polymerschicht (20) bestimmt wird und wobei zur Desorption des zu bestimmenden Gases in bestimmten Zeitabständen eine Erhitzung der Polymerschicht (20) auf eine gegenüber der Umgebungstemperatur erhöhte Temperatur erfolgt.

2. Verfahren nach Anspruch 1, **dadurch gekennzeichnet, dass** die Polymerschicht (20) ein Polymer mit basischen funktionellen Gruppen ist.

3. Verfahren nach Anspruch 2, **dadurch gekennzeichnet, dass** die Polymerschicht (20) ein Polyanilin umfasst.

4. Verfahren nach Anspruch 3, **dadurch gekennzeichnet, dass** als Polyanilin ein Copolymerisat von Anilin mit einer Aminobenzoesäure oder einer Aminobenzolsulfonsäure verwendet wird.

5. Verfahren nach einem der vorhergehenden Ansprüche, **dadurch gekennzeichnet, dass** die Bestimmung der elektrischen Leitfähigkeit, des elektrischen Widerstands oder der Impedanz der Polymerschicht (20) mittels mindestens zweier Elektroden (16, 18) erfolgt, die in Kontakt mit der Polymerschicht (20) stehen, wobei ein zwischen den Elektroden (16, 18) auftretender Stromfluss oder eine zwischen den Elektroden (16, 18) anliegende Spannung gemessen wird.

6. Verfahren nach einem der vorhergehenden Ansprüche, **dadurch gekennzeichnet, dass** die Polymerschicht (20) auf eine erhöhte Temperatur von 60 bis 400°C erhitzt wird.

7. Verfahren nach einem der vorhergehenden Ansprüche, **dadurch gekennzeichnet, dass** die Erhitzung der Polymerschicht (20) periodisch erfolgt.

8. Verfahren nach einem der vorhergehenden Ansprüche, **dadurch gekennzeichnet, dass** der Zeitraum t in Millisekunden, innerhalb dem die Polymerschicht (20) auf einer erhöhten Temperatur gehalten wird, nach der Formel $t = \exp(a/T)$ bestimmt wird, wobei a eine Zahl zwischen 3000 und 4000 ist und T die erhöhte Temperatur in Kelvin ist.

9. Sensor zur Bestimmung von sauren Gasen in Gasgemischen, wie insbesondere Chlorwasserstoff, Schwefeldioxid oder Stickstoffdioxid, mit einer Polymerschicht (20), die ihre elektrische Leitfähigkeit unter Einwirkung eines zu bestimmenden Gases verändert, mit mindestens zwei Elektroden (16, 18), die in Kontakt mit der Polymerschicht (20) stehen, und mit mindestens einem Heizelement (14) zur Erhitzung der Polymerschicht (20) auf eine gegenüber der Umgebungstemperatur erhöhte Temperatur, **dadurch gekennzeichnet, dass** ein Verfahren nach einem der Ansprüche 1 bis 8 durchführbar ist.

10. Sensor nach Anspruch 9, **dadurch gekennzeichnet, dass** zusätzlich ein Temperaturmesselement zur Bestimmung der Temperatur der Polymerschicht (20) vorgesehen ist.

11. Verwendung eines Verfahrens nach einem der Ansprüche 1 bis 8 oder eines Sensors nach einem der Ansprüche 9 und 10 zur Früherkennung von Bränden.

**10**

**16**          **18**

**20**

**12**

**14**

# Fig. 1

100M — a a' b b' c c' d d'    e e'    f f'    g g'

10M —

1M —

100K —

10K —

0        500      1000     1500     2000     2500     3000

# Fig. 2